# EUROPEAN PATENT APPLICATION

(11) **EP 2 036 567 A2**
(43) Date of publication of application: **18.03.2009**
(21) Application number: 08171779.5
(22) Date of filing: 06.12.2006
(51) Int. Cl.: A61K 31/7072, A61K 31/7076, A61K 38/05, A61K 38/06, A61K 38/07, A61K 38/08, A61K 38/10, A61P 9/08, A61P 9/10, A61P 9/12, A61P 9/04, A61P 15/10

(54) **Modulation of the P2Y2 receptor pathway**

(30) Priority: 06.12.2005 DK 200501729
(62) Divisional of application: 06818151.0
(71) Applicant: P2-Science APS, 2900 Hellerup (DK)
(72) Inventor: Rosenmeier, Jaya, DK-2900, Hellerup (DK)
(74) Representative: HOEIBERG A/S

(57) **Abstract**

The present invention relates to the field of regulating the activity of the purinergic receptors for the modulation of the vascular tone, particularly for the purpose of treatment of haemodynamic conditions by overriding of vasoconstriction activity, such as increases in sympathic vasoconstriction. Modulators, such as UTP analogues as described herein are preferably specific for P2Y2. Compounds capable of stimulating the P2Y2 receptor are suitable for the treatment or prevention wherein inhibition of vasoconstriction activity is desirable such as hypertension and hypertension relates disorders or diseases, whereas compound capable of counteracting the activity of the P2Y2 receptor are suitable for the treatment of or prevention wherein inhibition of vasodilatation is desirable.

## Description

All patent and non-patent references cited in the present application are hereby incorporated by reference in their entirety.

### Field of invention

The present invention relates to use of compounds capable of specifically modulating the P2Y2 receptor pathway in the treatment of diseases and to methods for identifying such compounds.

### Background of invention

The potent and widespread vascular actions of purine nucleotides and nucleosides have long been recognized. Extracellular nucleotides such as adenosine, AMP, ADP, ATP and UTP induce vasodilatation in human vessels by stimulating P2Y1, P2Y2/4 and P2Y6 receptors, while P2X receptor stimulation causes vascular smooth muscle contraction. These extracellular nucleotides are released continuously from various compartments such as red blood cells, platelets, leucocytes, endothelium, smooth muscle cells or neurons due to a variety of physiological stimuli such as decreases in oxygen tension, sheer stress, compression, damage or cell activation.

The purinergic receptors are distributed unevenly through the cardiovascular tree, but still very little is known about their full effect once activated and about where the individual receptors are located and how abundantly they are expressed in a given area or segment of the vascular tree. One of the potential effects of this system is to regulate blood pressure by altering vascular resistance, together with well known systems such as the autonomic nervous system and the renin-angiotensin system. The purinergic system can regulate vasomotor tone by inducing potent vasodilation through receptor activation on the endothelial side or vasoconstriction due to receptor activation on the vascular smooth muscle cells and thus control blood pressure in this manner.

That the purinergic receptors are vasodilators is well known (Folkow, 1949 Acta Physiol Scand 17, 311-316; Rongen et al. 1994 Circulation 90, 1891-1898; Ellsworth et al. 1995 Am J Physiol. 269:H2155-61.). However, to keep a vessel open by overriding the alfa-adrenergic receptor activation from the sympathetic nervous system is a pivotal capability of a metabolite that regulates vascular tone, especially when higher blood flows are needed in a setting of increased sympathetic drive such as during exercise or septic shock.

It has recently been demonstrated that circulating ATP when infused intraarterially into human skeletal muscle can increase blood flow and at the same time override increases in muscle sympathetic vasoconstrictor activity, thus simulating what occurs during exercise, a phenomenon termed functional sympatholysis (Rosenmeier et al. 2004 J. Physiol. 558:351-365).

### Summary of invention

It has now been found that it is the specific stimulation of the P2Y2 receptor that contributes to the local blood flow increase and blunts the augmented sympathetic vasoconstrictor activity. These observations have provided the basis for novel methods for treating haemodynamic conditions using compounds that specifically modulate activation of the P2Y2 receptor pathway. Furthermore, they have provided the basis for improved methods for screening for compounds that can be used in the treatment of haemodynamic conditions.

Thus, in a first main aspect, the invention relates to use of a compound capable of modulating the activation of the P2Y2 receptor pathway for the preparation of a medicament for the treatment of a haemodynamic condition in an individual in need thereof, wherein said compound
i) is capable of stimulating the human P2Y2 receptor, wherein the ratio of the EC50 for stimulation of the human P2Y2 receptor divided by the EC50 for stimulation of the human P2X1 receptor is higher, such as at least 2 fold higher, for said compound than the corresponding ratio for ATP, or
ii) is capable of counteracting stimulation of the human P2Y2 receptor.

By modulating the P2Y2 receptor pathway specifically, haemodynamic conditions can be treated more effectively, haemodynamic conditions that were difficult to treat can be treated and undesired side-effects can be reduced or avoided.

In an important main embodiment, the invention relates to the use of a compound capable of stimulating the P2Y2 receptor for the preparation of a medicament for increasing blood flow and reducing augmented sympathetic vasoconstrictor activity in an individual in need thereof, wherein the ratio of the EC50 for stimulation of the P2Y2 receptor divided by the EC50 for stimulation of the P2X1 receptor is higher, such as at least 2 fold higher, for said compound than the corresponding ratio for ATP.

Without being bound a specific theory, it is believed that compounds that have such a higher ratio as compared to ATP provide the same advantages as ATP in that they can increase blood flow and override increases in muscle sympathetic vasoconstrictor activity, but do not have, or have to a lesser degree, the disadvantages of ATP, i.e. the activation of purinergic P2X receptors, which results in vasoconstriction and risk of hypertension.

In another main embodiment, the reverse effect is envisaged in order to obtain vasoconstriction in situations where this is desired. Thus, in this embodiment, the invention relates to the use of compound capable of counteracting P2Y2 receptor stimulation for the preparation of a medicament for restoring sympathetic vasoconstrictor activity in an individual in need thereof.

In a further main aspect, the invention relates to a method for identifying a compound capable of increasing blood flow and reducing augmented sympathetic vasoconstrictor activity, said method comprising the steps of:
i) contacting a candidate compound with a functional P2Y2 receptor, and
ii) assessing in a quantitative manner whether or not said compound has a stimulating effect on said P2Y2 receptor.

In an even further aspect, the invention relates to a method for evaluating whether a compound is capable of increasing blood flow and reducing augmented sympathetic vasoconstrictor activity, said method comprising the steps of:
i) contacting a candidate compound with a functional P2Y2 receptor, and
ii) assessing in a quantitative manner whether or not said compound has a stimulating effect on said P2Y2 receptor.

### Description of Drawings

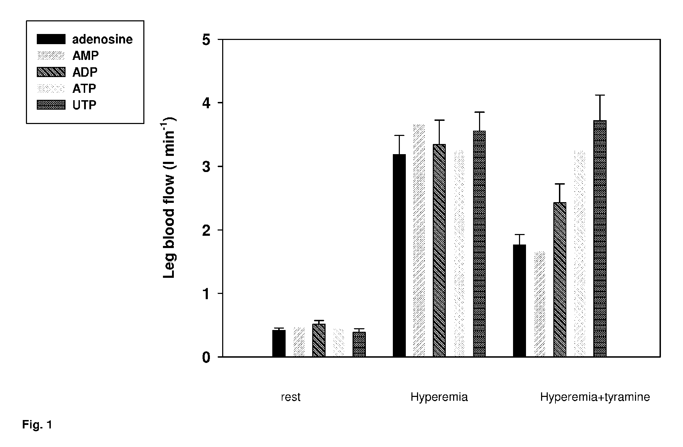

Fig 1: Blood flow responses to vasodilators and norepinephrine discharge by tyramine. Columns from left to right in each group represent: adenosine, AMP, ADP, ATP and UTP.

### Detailed description of the invention

### Definitions

When used herein, the term "modulation" is used for any direct or indirect change of receptor activation either in positive direction, e.g. stimulation, or in negative direction, e.g. blocking.

Unless specified otherwise, the term "treatment", when used herein, refers to all forms of treatment, such as curative treatment, ameliorating treatment, palliative treatment, or prophylactic treatment.

When used in connection with a receptor, the terms "stimulation" and "activation" indicate an interaction with said receptor that promotes the receptor to perform its normal biological function. For example, a compound capable of stimulating a G-protein coupled receptor can be a compound that through binding to said receptor promotes signal transduction via the G protein.

Unless specified otherwise, the term "receptor" refers to the human receptor.

The term "counteracting", when used herein in connection with a receptor, refers to an activity that directly or indirectly reduces the stimulation or activity of said receptor. A direct counteracting activity can e.g. arise when a compound antagonises or blocks a receptor by binding to it without activating it. An example of indirect counteracting of a receptor is a mechanism of reducing the formation or release of an agonist of said receptor. For example, by blocking the P2Y13 receptor, release of ATP can be reduced, thus counteracting the P2Y2 receptor pathway, which responds to ATP. Another example of indirect counteracting of a receptor is a mechanism whereby the downstream effects of a receptor are counteracted. For example, a P2X agonist can stimulate vasoconstriction, thus counteracting the vasodilatory effect of P2Y2 stimulation.

The term "hypertension", when used herein, refers to high blood pressure. This generally means that the systolic blood pressure is consistently over 140 and/or the diastolic blood pressure is consistently over 90. Either or both of these numbers may be too high.

When used herein, the term "septic shock" refers to a condition that occurs when an overwhelming infection leads to low blood pressure and low blood flow. Vital organs, such as the brain, heart, kidneys, and liver may not function properly or may fail. Decreased urine output from kidney failure may be one symptom.

"Cardiogenic shock" wherein used herein, refers to a disease state where the heart is damaged enough that it is unable to supply sufficient blood to the body.

"Ischemic heart disease" wherein used herein refers to a disease area constituting of every disease unit such as angina pectoris, unstable angina pectoris and acute myocardial infarction. In this disease the heart receives to little blood and thus the heart muscle itself is suffering, this may lead to cardiogenic shock.

### Embodiments

In a main embodiment of the invention, a compound capable of stimulating the P2Y2 receptor is used for the preparation of a medicament for increasing blood flow and reducing augmented sympathetic vasoconstrictor activity in an individual in need thereof, wherein the ratio of the EC50 for stimulation of the P2Y2 receptor divided by the EC50 for stimulation of the P2X1 receptor is at least 2 fold higher for said compound than the corresponding ratio for ATP.

In a preferred embodiment, the ratio of the EC50 for stimulation of the P2Y2 receptor divided by the EC50 for stimulation of the P2X1 receptor is at least 3 fold higher for the compound used than the corresponding ratio for ATP, more preferably, the ratio is at least 4 fold, e.g. at least 5 fold, such as at least 10 fold, e.g. at least 25 fold, such as at least 100 fold higher for the compound used than the corresponding ratio for ATP.

In preferred embodiments, the compound furthermore has an improved specificity as compared to ATP by having less stimulatory effect on one or more receptors selected from the group consisting of: P2X2, P2X3, P2X4, P2X5, P2X6 and P2X7.

Thus, in preferred embodiments:

The ratio of the EC50 for stimulation of the P2Y2 receptor divided by the EC50 for stimulation of the P2X2 receptor is at least 2 fold higher for said compound than the corresponding ratio for ATP, preferably at least 4 fold higher, e.g. at least 5 fold, such as at least 10 fold, e.g. at least 25 fold, such as at least 100 fold higher,
and/or
the ratio of the EC50 for stimulation of the P2Y2 receptor divided by the EC50 for stimulation of the P2X3 receptor is at least 2 fold higher for said compound than the corresponding ratio for ATP, preferably at least 4 fold higher, e.g. at least 5 fold, such as at least 10 fold, e.g. at least 25 fold, such as at least 100 fold higher,
and/or
the ratio of the EC50 for stimulation of the P2Y2 receptor divided by the EC50 for stimulation of the P2X4 receptor is at least 2 fold higher for said compound than the corresponding ratio for ATP, preferably at least 4 fold higher, e.g. at least 5 fold, such as at least 10 fold, e.g. at least 25 fold, such as at least 100 fold higher,
and/or
the ratio of the EC50 for stimulation of the P2Y2 receptor divided by the EC50 for stimulation of the P2X5 receptor is at least 2 fold higher for said compound than the corresponding ratio for ATP, preferably at least 4 fold higher, e.g. at least 5 fold, such as at least 10 fold, e.g. at least 25 fold, such as at least 100 fold higher,
and/or
the ratio of the EC50 for stimulation of the P2Y2 receptor divided by the EC50 for stimulation of the P2X6 receptor is at least 2 fold higher for said compound than the corresponding ratio for ATP, preferably at least 4 fold higher, e.g. at least 5 fold, such as at least 10 fold, e.g. at least 25 fold, such as at least 100 fold higher,
and/or
the ratio of the EC50 for stimulation of the P2Y2 receptor divided by the EC50 for stimulation of the P2X7 receptor is at least 2 fold higher for said compound than the corresponding ratio for ATP, preferably at least 4 fold higher, e.g. at least 5 fold, such as at least 10 fold, e.g. at least 25 fold, such as at least 100 fold higher.

### Compounds capable of stimulating the P2Y2 receptor

In a main embodiment, the compound used in the invention is a compound capable of stimulating the P2Y2 receptor. Preferably the stimulation is direct, i.e. said compound acts as an agonist.

In a preferred embodiment, the compound used has a serum half-life in humans of at least 10 min, such as at least 30 min, e.g. at least 1 hour, such as at least 3 hours. Serum half-life can be assessed by methods well known to the person skilled in the art.

In an embodiment the compound used in the invention is a compound of formula (I): 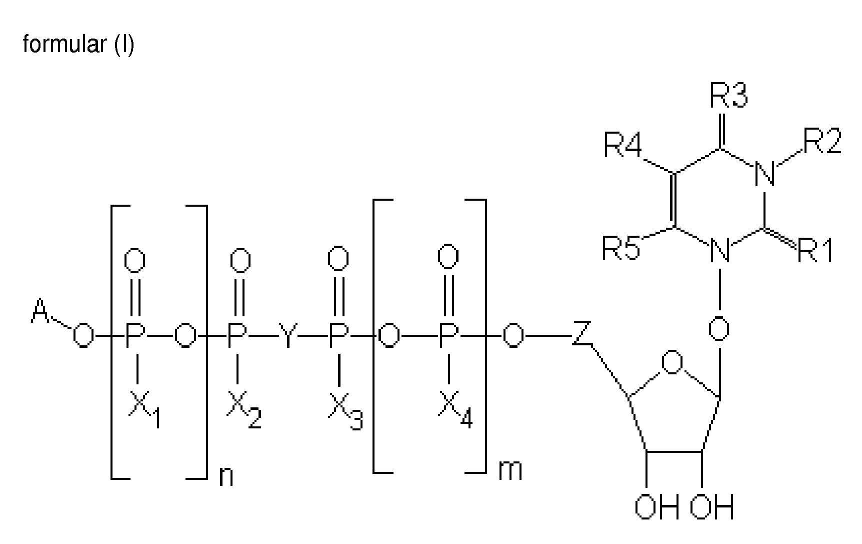 wherein
R1 is O, S, hydroxyl, mercapto, amino or cyano,
R2 is H, Br, nothing, acyl, C1-6 alkyl or sulphonate,
R3 is O, S, hydroxyl, mercapto or amino,
R4 is H, hydroxyl, methyl, cyano, nitro, halogen such as Br,
R5 is H or Br
X₁, X₂, X₃ and X₄ are independently O⁻ or S⁻,
Y is O, imido, methylene or dihalomethylene, such as difluromethylene,
Z is CH₂,
n and m are independently 0 or 1, and n+m is 0,1 or 2,
A is H or
ribose, linked at the 5 position with a pyrimidine or purine residue or pyrimidine or purine derivative selected from the group of uracil, cytosine, guanine, adenine, xanthine, hypoxanthine, linked through the 1 or 9 position, respectively or
   ribose linked at the 5 postion with a pyrimidine residue having formular II 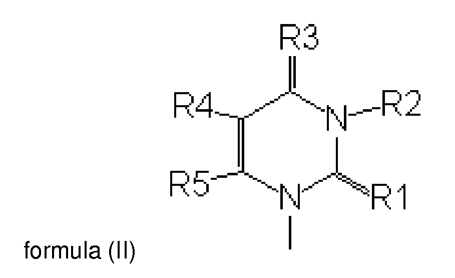 wherein the denoted R groups are as listed above or
   ribose lined at the 5 position with a purine residue having formular (III) 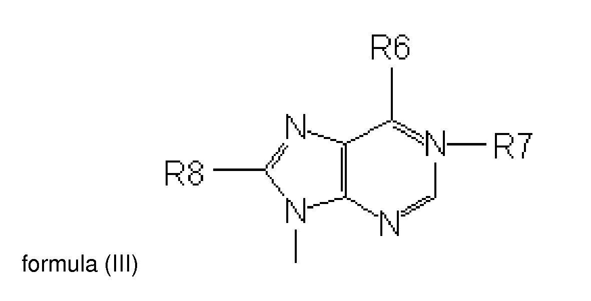 wherein R6 is NH2, while R7 is nothing and there is a double bond between N1 and C6 (adenine) or
   wherein R6 is NH2 and R7 is O and there is a double bound between N1 and C6 (adenine-1-oxide) or
   wherein R6 and R7 together forma a ring of -NCH=CH- from N6 to N1 with a double bound between N-6 and C6 (1, N⁶-ethenoadenine),

In a different embodiment the compound used in the invention is a compound of formula (I): 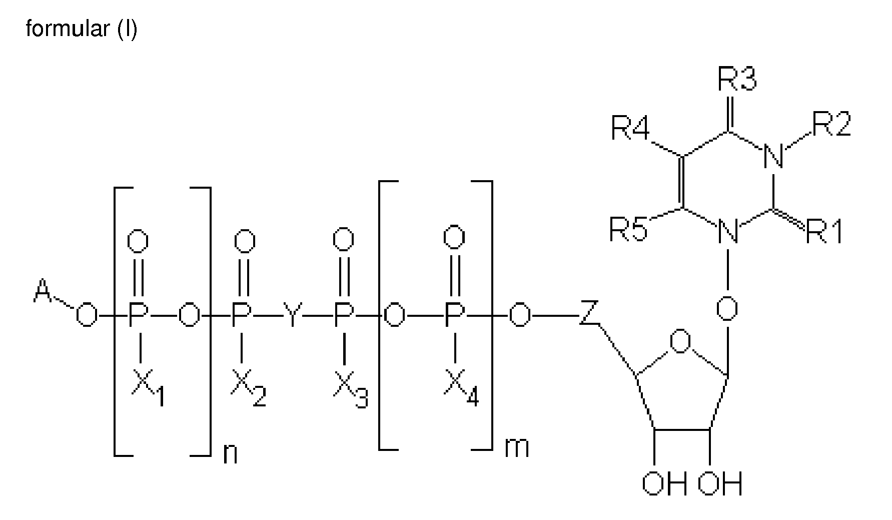 wherein
R1 is O, S,
R2 is H, Br or nothing
R3 is O, S, or NH₂,
R4 is H, Br or methyl,
R5 is H or Br
X₁, X₂, X₃ and X₄ are independently O⁻ or S⁻,
Y is O, imido, methylene or dihalomethylene, such as difluromethylene,
Z is CH₂,
n and m are independently 0 or 1, and n+m is 0,1 or 2,
A is H or
   ribose, linked at the 5 position with a pyrimidine or purine residue or pyrimidine or purine derivative selected from the group of uracil, cytosine, guanine, adenine, xanthine, hypoxanthine, linked through the 1 or 9 position, respectively.

In a subsequent embodiment the compound is a P2Y2 agonist of formula (I) wherein Y is O.

In a subsequent embodiment the compound is a P2Y2 agonist of formula (I) wherein A is H.

In an embodiment the compound is a P2Y2 agonist of formula (I) wherein Y is O and A is H.

In an embodiment the compound is of formula I, wherein n + m = 1.

In a specific embodiment, the compound used is UTP. In another embodiment, the compound used is different from UTP.

In an embodiment the compound is of formula I, wherein n + m = 2.

In a further embodiment, the compound used is P¹-(uridine 5')-P⁴-(2'-deoxycytidine 5')tetraphosphate or a salt thereof, such as a tetrasodium salt (INS37217).

In an embodiment the compound is of formula I, wherein n + m = 2, such as a modified UDP molecule.

In a yet further embodiment, the compound used is one of the compounds described in US 5,292,498. Accordingly, in a preferred embodiment, the compound used is a compound of the formula (I): 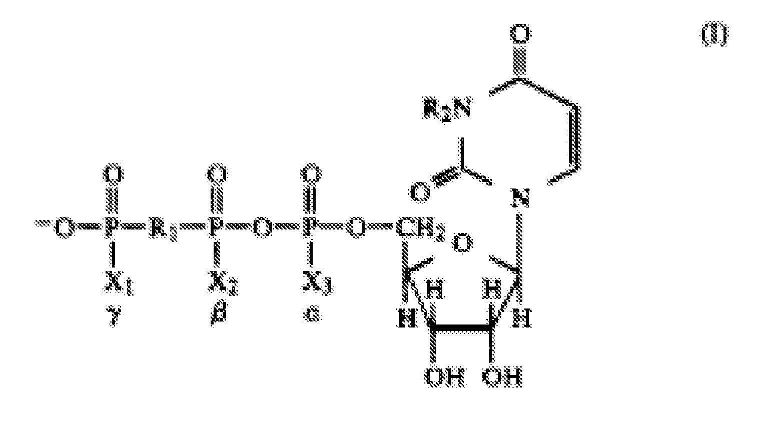
wherein X₁, X₂, and X₃ are each independently either O⁻ or S⁻,
and R₁ is O, imido, methylene, or dihalomethylene,
and R₂ is H or Br.

In another embodiment, the compound used is one of the P2Y2 agonists described in US 5,789,391, Thus, in such an embodiment, the compound used is a compound of the formula (VII): 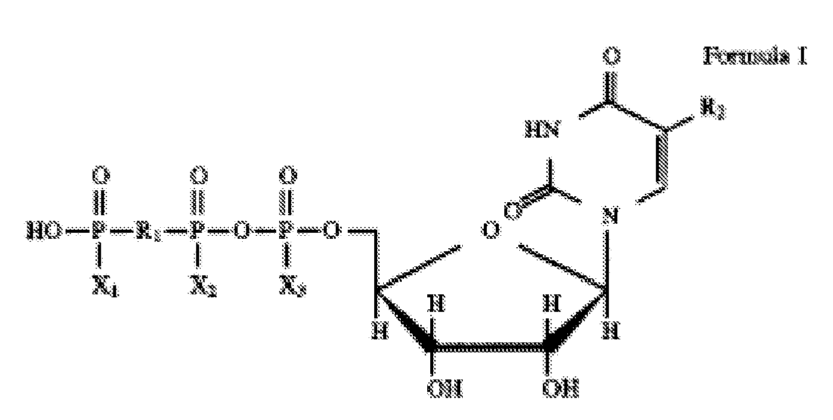
wherein X₁, X₂, and X₃ are each independently either O⁻ or S⁻,
and R₁ is O, imido, methylene, or dihalomethylene,
and R₂ is H or Br.

In another embodiment, the compound used is a compound of the formula (VI): 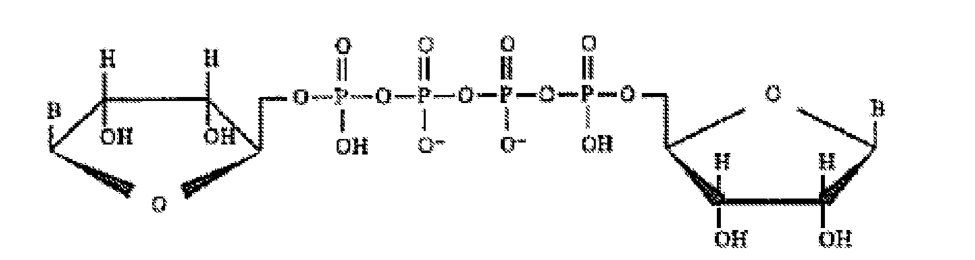 wherein B is independently: 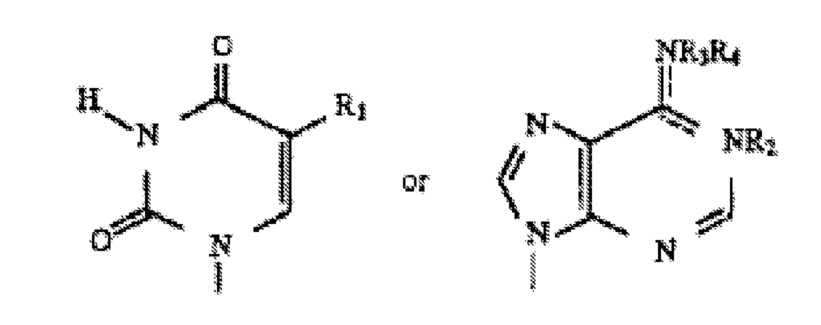 wherein:
R₁ is selected from the group consisting of H and Br;
R₃ and R₄ are H while R₂ is nothing and there is a double bond between N-1 and C-6 (adenine); or
R₃ and R₄ are H while R₂ is O and there is a double bond between N-1 and C-6 (adenine-1-oxide); or
R₃, R₄ and R₂ taken together are -CH=CH-, forming a ring from N-6 to N-1 with a double bond between N-6 and C-6 (1,N⁶-ethenoadenine).

In yet another embodiment, the compound used is one of the P2Y2 agonists described in US 5,837,861, Thus, in such an embodiment, the compound used is a compound of the formula (II): 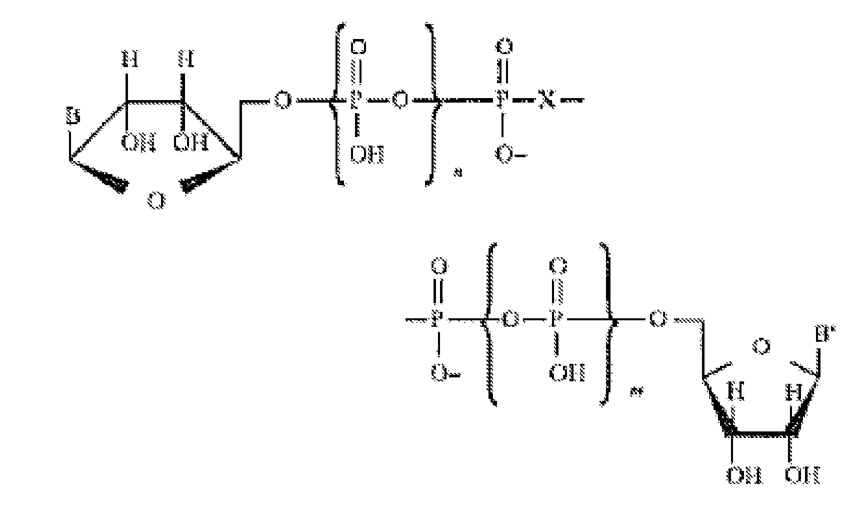
wherein X is oxygen, methylene or difluoromethylene,
and n=0 or 1
and m= 0 or 1
and n+m = 0,1 or 2
and B and B' are each independently a purine residue, as in Formula III, or a pyrimidine residue, as in Formula IV, linked through the 9- or 1- position respectively: 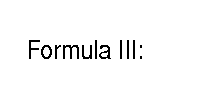 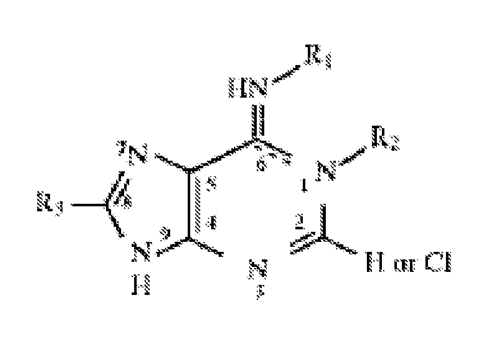 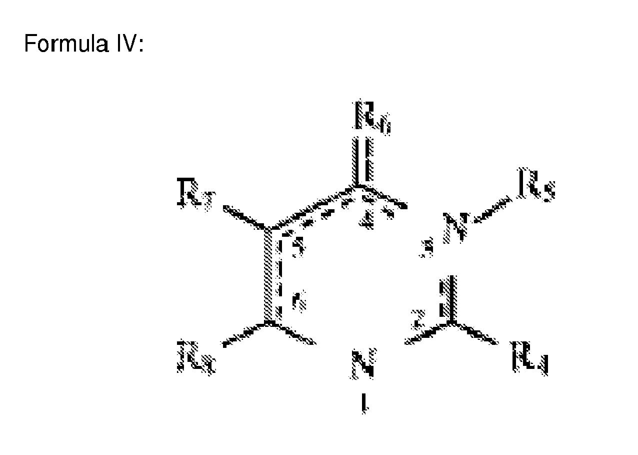 wherein: R₂ is O or is absent; or
   R₁ and R₂ taken together form an optionally substituted 5-membered raised imidazole ring, or
   R₁ and/or R₃ are chosen from the group consisting of:
   (a) arylalkyl (C1-6) groups with the aryl moiety optionally substituted,
   (b) alkyl,
   (c) ([6-aminohexyl]carbamoylmethyl),
   (d) ω-amino alkyl (C2-1 0),
   (e) ω-hydroxy alkyl (C2-10),
   (f) ω-thiol alkyl (C2-10),
   (g) ω-carboxy alkyl (C2-10),
   (h) the ω-acylated derivatives of (b), (c) or (d) wherein the acyl group is either acetyl, trifluroroacetyl, benzoyl, or substituted-benzoyl alkyl (C2-10), and
   (i) ω-carboxy alkyl (C2-1 0) as in (e) above wherein the carboxylic moiety is an ester or an amide;
and wherein R₄ is hydroxy, mercapto, amino, cyano, aralkoxy, C1-6 alkylthio, C1-6 alkoxy, C1-6 alkylamino or dialkylamino, wherein the alkyl groups of said dialkylamino are optionally linked to form a heterocycle;
and wherein R₅ is hydrogen, acyl, C1-6 alkyl, aroyl C1-5 alkanoyl, benzoyl, or sulphonate,
and wherein R₆ is hydroxy, mercapto, alkoxy, aralkoxy, C1-6 -alkylthio, C1-5 disubstituted amino, triazolyl, alkylamino or diamino, wherein the alkyl groups of said dialkylamino are optionally linked to form a heterocycle or linked to N³ to form am optionally substituted ring;
or R₅ -R₆ together forms a 5 or 6-membered saturated or unsaturated ring bonded through N or O at R₆, wherein said ring is optionally substituted;
and wherein R₇ is selected from the group consisting of:
(a) hydrogen,
(b) hydroxy,
(c) cyano,
(d) nitro,
(e) alkenyl, wherein the alkenyl moiety is optionally linked through oxygen to form a ring optionally substituted with alkyl or aryl groups on the carbon adjacent to the oxygen,
(f) substituted alkynyl
(g) hydrogen
(h) halogen,
(i) alkyl,
(j) substituted alkyl,
(k) perhalomethyl,
(l) C2-6 alkyl,
(m) C2-3 alkenyl,
(n) substituted ethenyl,
(o) C2-3 alkynyl and
(p) substituted alkynyl when R₆ is other than amino or substituted amino;
   and wherein R₈ is selected from the group consisting of:

(a) hydrogen,
(b) alkoxy,
(c) arylalkoxy,
(d) alkylthio,
(e) arylalkylthio,
(f) carboxamidomethyl,
(g) carboxymethyl,
(h) methoxy,
(i) methylthio,
(j) phenoxy and
(k) phenylthio.

Specific examples of such compounds are:
- P1,P4-Di(uridine 5'-P2,P3-methylene tetraphosphate)
- P1,P4-Di(uridine 5'-P2,P3-difluoromethylenetetraphosphate)
- P1.P4-Di(uridune 5'-P2,P3-imidotetraphosphate
- P1,P4-Di(4-thioruridine 5'-tetraphosphate)
- P1,P5-Di(uridine 5'-pentaphosphate)
- P1,P4-Di(3,N⁴-ethenocytidine 5'-tetraphosphate)

In a further embodiment, the compound used is one of the P2Y2 agonists described in US 2002/0082417. Accordingly, in one embodiment, the compound used is a compound of the formula (V): 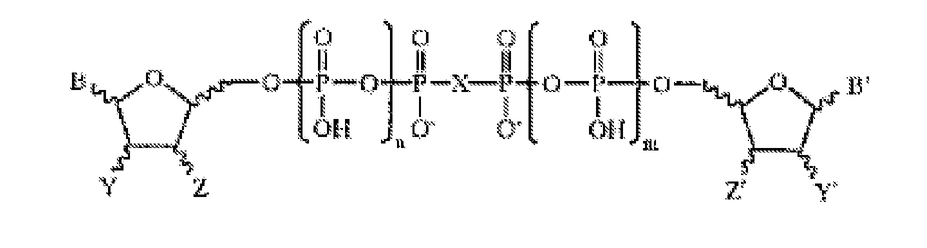 wherein:
X is oxygen, methylene, difluoromethylene, imido;
n=0, 1 or 2;
m=0, 1 or 2;
n+m=0, 1, 2, 3 or 4; and
B and B' are each independently a purine residue or a pyrimidine residue linked through the 9- or 1- position, respectively;
Z=OH or N₃;
Z'=OH or N₃;
Y=H or OH;
Y'=H or OH;

In a preferred embodiment, Z is N₃ when Y is H or Z' is N₃ when Y' is H.

Preferred compounds of formula V are the compounds of formula Va: 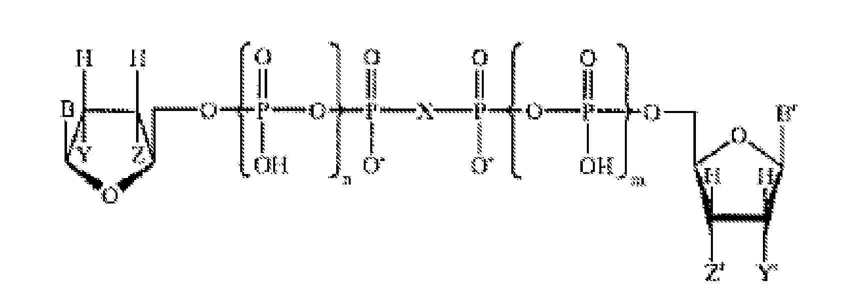 wherein
X=O;
n+m=1 or 2;
Z, Z',Y and Y'=OH;
B and B' are uracil, thymine, cytosine, guanine, adenine, xanthine, hypoxanthine or as defined in Formulas III and IV; or
X=O;
n+m=3 or 4;
Z, Z', Y and Y'=OH;
B=uracil;
B' is uracil, thymine, cytosine, guanine, adenine, xanthine, hypoxanthine or as defined in Formulas III and IV; or
X=O;
n+m=1 or 2;
Z, Y and Z'=OH;
Y'=H;
B=uracil;
B' is uracil, thymine, cytosine, guanine, adenine, xanthine, hypoxanthine or as defined in Formulas III and IV; or
X=O;
n+m=0, 1 or 2;
Z and Y=OH;
Z'=N₃;
B=uracil;
B'=thymine; or
X=O;
n+m=0, 1 or 2;
Z and Z'=N₃;
Y and Y'=H;
B and B'-thymine; or
X=CH2, CF2 or NH;
n and m=1;
Z, Z', Y and Y'=OH;
B and B' are uracil, thymine, cytosine, guanine, adenine, xanthine, hypoxanthine or as defined in Formulas III and IV above.

In yet another embodiment, the compound used is one of the compounds that bind the P2Y2 receptor described in US2004/0116339. Thus, in a preferred embodiment, the compound used is a peptide selected from the group consisting of:
YARGDHWPFST (SEQ ID NO:1),
SVRGTRITCHDTSARELFSHFVAY (SEQ ID NO:2),
LYYSFRSLDLSCHTLNAINMAYKITR (SEQ ID NO:3),
AAADLEPWNSTINCTWELDELCYKCRFNEDFKYVL (SEQ ID NO:4),
Ala-Ala-Ala-Ala-Glu (SEQ ID NO:5),
Ala-Gly-Ala-Ala (SEQ ID NO:6),
Ala-D-Ala-D-Ala-D-Ala-D-Ala-D-Ala (SEQ ID NO:7),
Gly-Gly-Ser-Ala (SEQ ID NO:8),
Gly-Gly-Gly (SEQ ID NO:9),
Gly-Gly-Gly-Ala (SEQ ID NO:10),
Gly-Gly-His-Gly (SEQ ID NO:11),
Gly-Ala-Ala-D-Ala-L-Ala (SEQ ID NO:12),
Gly-Gly-Ala-Gly (SEQ ID NO:13),
Gly-Ala-Ala (SEQ ID NO:14),
Ser-Glu-Gly (SEQ ID NO:15),
Gly-Gly-Glu-Ala (SEQ ID NO:16),
Glu-His-Gly (SEQ ID NO:17),
Gly-Ala-Asn (SEQ ID NO:18),
Ala-Ala-Ala-L-Pro (SEQ ID NO:19),
D-Ala-Ala-Ala-Ala (SEQ ID NO:20),
His-Gly-Gly (SEQ ID NO:21),
D-Glu-Glu (SEQ ID NO:22),
Gly-Gly-His-Ala (SEQ ID NO:23),
Lys-Gly-Glu (SEQ ID NO:24),
D-Glu-D-Glu (SEQ ID NO:25),
Gly-Pro-Ala (SEQ ID NO:26),
Ala-Ala-Tyr (SEQ ID NO:27),
Glu-Glu-Asp-OH (SEQ ID NO:28),
Gly-Gly-beta-Ala-Gly (SEQ ID NO:29),
AAsp-Ala-Ser-Gly-Glu (SEQ ID NO:30),
Glu-Glu-Gln (SEQ ID NO:31),
Ala-Ala-Pro-Ala (SEQ ID NO:32),
Gly-Gly-Tyr-Ala (SEQ ID NO:33),
Trp-Gly-Gly-Tyr (SEQ ID NO:34),
Glycyl-L-alanyl-L-glutamine (SEQ ID NO:35),
S-Nitrosoglutathione (SEQ ID NO:36),
Glutathione-monoisopropyl-ester (reduced) (SEQ ID NO:37),
L-Cystinyl-cystine (SEQ ID NO:38),
Ac-His-His-Gly-His (SEQ ID NO:39),
Gly-Ala-NH2 (SEQ ID NO:40),
Ac-Glycyl-L-Glutamine (SEQ ID NO:41),
Methoxycarbonyl-Phe-Gly (SEQ ID NO:42), and
3-5-Diiodo-D-Tyr-Ala-Gly-Gly (SEQ ID NO:43).

In an even further embodiment, the compound is selected from the group consisting of: P-nitrobenzyl-oxycarbonyloxy-succinimide; 1-benzyl-2-2-5-dioxotetrahydro-1H-pyrrol-1-yl-pyrrolidine-1-2-dicarboxylate; 5-3-methoxycarbonyl-acryloyl-isophthalic-acid-dimethyl-ester; 6-O-[beta]-glucopyranosyl-[beta]-D-glucose; cyclobutane-1-2-3-4-tetracarboxylic-acid-dimethyl-ester, mono-2-acryloyloxyethyl-succinate; 2-benzyloxycarbonylamino-3-methyl-butyric-acid-2-5-dioxo-pyrrolidin-1-yl-ester; tris trichlorosilylethylmethylsilane; 2-5-dioxotetrahydro-1H-pyrrol-1-yl-2-benzyloxycarbonylamino-3-phenylpropane; 2-chloro-ethyl-carbamic-acid-2-5-dioxopyrrolidin-1-yl-ester; mono-2-methacryloyloxyethyl-succinate; galactosyl-diglyceride; 3-beta-hydroxy-5-alpha-androstan-17-one-beta-D-glucoside; N-4-5-dimethoxy-2-nitrobenzyloxycarbonyl-L-tryptophan; 2-amino-2-4-chlorobenzoyloxyiminoethyl-pivalate; disuccinimidyl-sebacate; 2-2-2-trichloro-1-2-methoxy-phenylamino-ethyl-carbamic-acid-benzyl-ester; 2-acetamido-2-deoxy-6-O-beta-D-galactopyranosyl-D-galactopyranose; 2-benzyloxycarbonylamino-3-hydroxy-propionylamino-acetic-acid-ethyl-ester; 2-benzyloxycarbonylmethylsulfanyl-6-ho-pyrimidine-4-carboxylic-acid-methyl-ester; 3-3-2-2-2-trichloro-1-isobutyrylamino-ethyl-thioureido-benzoic-acid; ester-of-2-diazo-1-naphthol-5-sulphone-with-2-3-4-trihydroxybenzophenone; 4-5-dihydroxy-6-hydroxymethyl-2-meo-4h-pyran-3-yl-carbamic-acid-benzyl-ester; diethyl-trans-1-2-cyclopropanedicarboxylate; 2-allyloxycarbonylmethylsulfanyl-6-HO-pyrimidine-4-carboxylic-acid-methyl-ester; 1-O-alpha-D-glucopyranosyl-D-fructose; methyl-2-2-4-chlorophenylsulfonylamino-acetylamino-2-phenylacetate; N+4-2-hydroxy-4-oxo-4-phenylbut-2-enoyl-2-methylquinoline-4-carbohydrazide; or DL-Djenkolic-acid.

### Assays

Assays for P2Y2 activity can be performed in several ways described in the art. A suitable example of an assay is the assay described in Lazarowski et al. (1995) Brit. J. Pharm. 116,1619-1627. Another example is the assay described in Erb (1995) J. Biol. Chem. 270:4185-4188. A number of further examples of suitable assays is given in Müller (2002) Curr. Pharm. Design 8:2353-2369. Particularly suitable are the assays described in Parr et al. (1994) Proc. Natl. Acad. Sci USA 91:3275-3279 and in Velazquez et al. (2000) Mol. Cell. Biochem. 206:75-89. In order to allow a proper comparison with ATP, it is normally preferred that the EC50 for the compound used in the invention is compared with the EC50 for ATP in the same assay.

In a further main aspect, the invention relates to an ex vivo or in vivo method for identifying a compound capable of increasing blood flow and reducing augmented sympathetic vasoconstrictor activity, said method comprising the steps of:
i) contacting a candidate compound with a functional P2Y2 receptor, and
ii) assessing whether or not said compound has a stimulating effect on said P2Y2 receptor.

In another main aspect, the invention relates to an ex vivo or in vivo method for evaluating whether a compound is capable of increasing blood flow and reducing augmented sympathetic vasoconstrictor activity, said method comprising the steps of:
i) contacting a candidate compound with a functional P2Y2 receptor, and
ii) assessing whether or not said compound has a stimulating effect on said P2Y2 receptor.

In a preferred embodiment of the above methods, the method used is one of the methods described above under "Assays." More preferably, the method used is the one described in Velazquez et al. (2000) Mol. Cell. Biochem. 206:75-89, i.e. a cell-based assay in which 132N1 astrocytoma cells stably transfected with the human P2Y2 receptor are brought in contact with the compound to be tested and the mobilization of intracellular calcium is assayed.

### Activation of P2X receptors

As disclosed above, in a main embodiment of the invention, a compound capable of stimulating the P2Y2 receptor is used for the preparation of a medicament for increasing blood flow and reducing augmented sympathetic vasoconstrictor activity in an individual in need thereof, wherein the ratio of the EC50 for stimulation of the P2Y2 receptor divided by the EC50 for stimulation of the P2X1 receptor is at least 2 fold higher for said compound than the corresponding ratio for ATP.

The capacity of compound to activate the P2X1 receptors or any of the receptors selected from the group consisting of P2X2, P2X3, P2X4, P2X5, P2X6 and P2X7 can be determined in several ways described in the art. For example, a number of suitable references has been provided in North (2002) Physiol. Rev. 82:1013-1067. An example of a suitable assay is the one described in Evans et al. (1995) Mol Pharmacol. (2):178-83. Thus, in a preferred embodiment, the method used is a cellular assay based on recombinant human cells, preferably oocytes or HEK 293 cells, that express a P2X receptor, e.g. the P2X1 receptor from human bladder smooth muscle. In order to allow a proper comparison with ATP, it is normally preferred that the EC50 for the compound used in the invention is compared with the EC50 for ATP in the same assay.

### Compounds capable of counteracting the P2Y2 receptor

In a different embodiment, the invention relates to the use of a compound capable of counteracting P2Y2 receptor stimulation for the preparation of a medicament for restoring sympathetic vasoconstrictor activity in an individual in need thereof, preferably an individual suffering from, or being at risk of developing, haemodynamic shock, such as septic shock or cardiogenic shock.

In a preferred embodiment, said compound is a compound that blocks, i.e. antagonises, the P2Y2 receptor.

In one such embodiment, the compound used is one of the compounds described in US 20050181503. Accordingly, in one embodiment, the compound is selected from the group consisting of suramin, periodate oxidized adenosine 5'-triphosphate ("Oxidized-ATP"), brilliant blue G ("BBG"), hexamethylene amiloride ("HMA"), diinosine pentaphosphate ("Ip5I"), pyridoxal-5'-phosphate-6-azophenyl-2',5'-disulphonic acid ("isoPPADS"), 1-[N,O-bis(5-isoquinolinesulfonyl)-N-methyl-L-tyrosyl]-4-phenyl-piperazine ("KN-62"), pyridoxal-5'-phosphate-6-azophenyl-4'-carboxylate ("MRS 2159"), 8,8'-(carbonylbis(imino-3,1-phenylenecarbonylimino)bis(1,3,5-napththalene-trisulfonic acid) ("NF023"), 8,8'-(carbonylbis(imino-4,1-phenylenecarbonylimino-4,1-phenylenecarbonylimino)bis(1,3,5-napththalenetrisulfonic acid) ("NF279"), pyridoxal-5'-phosphate-6-(2'-naphthylazo-6-nitro-4',8'-disulphonate) ("PPNDS"), reactive blue 2 ("RB-2"), 2',3'-O-(2,4,6-trinitrophenyl) adenosine triphosphate ("TNP-ATP"), adenosine 3'-phosphate 5'-phosphosulphate ("A3P5PS"), 2'-deoxy-N-6-methyladenosine-3',5'-bisphosphate ("MRS 2179"), (N)-methanocarba-N-6-methyl-2-chloro-2'-deoxy-adenosine-3',5'-bisphosphate ("MRS 2279"), pyridoxal-5'-phosphate-6-azophenyl-2',4'-disulphonic acid ("PPADS"), N6-[2-(methylthio)-ethyl]-2-(3,3,3-trifluoropropyl)thio-5'-adenylic acid ("AR-C69931MX"), N1-(6-ethoxy-1,3-benzothiazol-2-yl-2-(7-ethoxy-4-hydroxy-2,2-dioxo-2H-2-6benzo[4,5][1,3]thiazolo[2,3-c]-[1,2,4]thiadiazin-3-yl)-2-oxo-1-ethanesulfonamide ("C1330-7"), 2-methylthioadenosine-5'-monophosphate ("2-MeSAMP"), 8-cyclopentyl-1,3-dimethylxanthine ("CPT"), 8-cyclopentyl-1,3-dipropylxanthine ("CPX"), 3-(3-Iodo-4-aminobenzyl)-8-(4-oxyacetate)-phenyl-1-propyl xanthine ("I-ABOPX"), 1,3-diethyl-8-(3,4-dimethoxyphenylethyl)-7-methyl-3-,7-dihydro-1H-purine-2,6-dione ("KW 6002'), 3-ethyl 5-benzyl 2-methyl-6-phenyl-4-phenylethynyl-1,4-(.+-.)-dihydropyridine-3,5-dicarbox- ylate ("MRS 1191 "), 2,3-diethyl-4,5-dipropyl-6 - phenylpyridine-3-thiocarbo- xylate-5-carboxylate ("MRS 1523"), 9-chloro-2-(2-furyl)-5-phenylacetylamin-o[1,2,4]-triazolo[1,5-c]quinazoline ("MRS 1220"), N6-cyclopentyl-9-methyladenine ("N-0840"), N-(2-methoxyphenyl)-N'-(2-(3-pyridyl)quinazolin-4-yl)urea ("VUF 5574"), 8-(N-methylisopropyl)amino-N-(5'-endohydroxy-endonorbornyl)-9-methyladenine ("WRC-0571 "), 8-[4-[[[[(2-aminoethyl)amino]carbonyl]methyl]oxy]phenyl]-1,3-dipropylxanthine, xanthine amine congener ("XAC"), 8-[4-[[(4-cyano)phenylcarbamoylmethyl]oxy]phenyl]-1,3-di-(n-propyl)xanthine ("MRS 1754"), 8-(3-chlorostyryl)caffeine, and alloxazine.

In another embodiment, the compound capable of counteracting the P2Y2 receptor is a compound that reduces ATP discharge from red blood cells.

In one such embodiment, the compound is a compound that blocks, i.e. antagonises, the P2Y13 receptor. Examples of such compounds are derivatives of PPADS (pyridoxal-5'-phosphate-6-azo-phenyl-2,4-disulfonate), described in Kim et al. (2005) Biochem. Pharmacol. 70:266-74, such as analogues of PPADS modified through substitution of the phenylazo ring, including halo and nitro substitution, and 5'-alkyl phosphonate analogues. Preferred are 6-(3-nitrophenylazo) derivatives of pyridoxal-5'-phosphate, the 2-chloro-5-nitro analogue (MRS 2211) and the 4-chloro-3-nitro analogue (MRS 2603). Other examples of compounds are NF023, TNP-ATP, suramin, PPADS, DIDS (4,4'-diisothiocyanatostilbene-2,2'-disulfonic acid), Ip5I and PPNDS. The compound AR-C67085 is a further example of a preferred compound (Wang (2005) Circ Res. 96(2):189-96). P2Y13 antagonists can be identified as described in US 6,946,244.

In a further embodiment, the compound used is a P2X agonist.

### Treatment

Cardiovascular diseases are commonly regulated by the sympathetic nervous systems ability to cause dilation or constriction.

By specific modulation of the P2Y2 receptor regulation of the sympathetic nervous systems ability to cause dilation or constriction without targeting the sympathetic nervous system directly, may be obtained.

### Clinical indications - conditions to be treated with a P2Y2 agonist

In an embodiment of the invention the clinical indication to be treated may be treated by administration of a P2Y2 agonist capable of stimulating the P2Y2 receptor, preferably with a high specificity compared to the stimulatory effect on P2X receptors. The activation of P2Y2 may lead to stimulation of blood flow and/or inhibition of vasoconstriction activity and thereby meliorate haemodynamic conditions

A haemodynamic conditions may affect an individual in one or more local section of the body.

The human or animal body may be divided into the sections head, neck, torso and limbs. In an embodiment the individual being treated is suffering from or at risk of developing a haemodynamic conditions affecting a sections of the body selected from the group of; head, neck and torso.

The head section comprising skull, forehead, eyes, ears, nose, mouth, teeth, tongue, jaw, face, cheek and chin. In an embodiment the individual being treated is suffering from or at risk of developing a haemodynamic conditions affecting a section of the body selected from the group of: forehead, eyes, ears, nose, mouth, tongue, face, cheek and chin, or in a further embodiment in a section of the body selected from the group of: forehead, ears, nose, mouth, tongue, face, cheek and chin.

The neck section includes the regions; throat, Adam's apple and larynx.
In an embodiment the individual being treated is suffering from or at risk of developing a haemodynamic conditions affecting a section of the body selected from the group of: throat, Adam's apple and larynx

The sections of the torso include shoulders, spine, chest, breast, ribcage, abdomen and belly button, sex organs (penis/scrotum/testicle/clitoris/vagina/ovary/uterus), hip, anus and buttocks. In an embodiment the individual being treated is suffering from or at risk of developing a haemodynamic conditions affecting a section of the body selected from the group of: shoulders, spine, chest, breast, ribcage, abdomen and belly button, sex organs, hip, anus and buttocks.

The limbs are external body part, or natural prolongation, that protrudes from the human or animal body such, as arm, elbow, forearm, wrist, hand, finger (thumb/Index/middle/ring/little), leg, lap, thigh, knee, calf, heel, ankle, foot and toe (Hallux).

In an embodiment the individual being treated is suffering from or at risk of developing a haemodynamic conditions affecting a section of the body selected from the group of: arm, elbow, forearm, wrist, hand, finger, leg, lap, thigh, knee, calf, heel, ankle, foot and toe (Hallux).

In a preferred embodiment the individual being treated is suffering from or at risk of developing a haemodynamic conditions affecting a section of the body selected from the group of: arm, elbow, forearm, wrist, hand and finger, In an alternative preferred embodiment the individual being treated is suffering from or at risk of developing a haemodynamic conditions affecting a section of the body selected from the group of: leg, lap, thigh, knee, calf, heel, ankle, foot, toe (Hallux).

### Hypertension

In one embodiment of the use of the invention, the individual being treated is suffering from, or at risk of developing hypertension, such as arterial hypertension, including essential (primary) or secondary hypertension. Further included is pulmonary hypertension, including primary pulmonary hypertension wherein pulmonary hypertension is characterized by an elevated blood pressure in the pulmonary circulation. One of the features of primary pulmonary hypertension (PPH), a human condition of unknown aetiology is increased pulmonary vascular resistance. Artificial stimulation of the P2Y2 receptor can reduce the pulmonary wedge pressure and restore the pressure in the lungs to normal values, thus avoiding right heart failure that results in very premature death (mean survival time after diagnosis ∼5 years).

In an embodiment the individual being treated is suffering from or at risk of developing pregnancy-induced hypertension, hypertensive retinopathy, hypertensive heart disease, renovascular hypertension, hypertensive nephropathy, malignant hypertension, hypertensive emergency, portal hypertension with an elevated blood pressure in the portal vein or portocaval system and white coat hypertension, when blood pressures are elevated in a clinical setting but not when measured the patient at home.

In a further different embodiment the individual being treated is suffering from or at risk of developing intracranial hypertension (e.g. benign intracranial hypertension) that is unrelated to blood pressure but refers to increased pressure inside the cranial vault of the skull.

In an embodiment the individual being treated is suffering from or at risk of developing orthostatic hypotension (also known as postural hypotension and, colloquially, as head rush).

In some situations hypertension may result as a secondary effect from other treatment or as a symptom of other diseases. In another embodiment of the use of the invention, the individual to be treated is an individual suffering from pheochromocytoma. Patients suffering from pheochromocytoma have tumors that produce norepinephrine. The vasoconstriction caused by this compound can be overridden by treatment with the compounds defined in the invention. In another embodiment of the invention, the individual to be treated is an individual undergoing chemotherapy, such as anti-neoplastic therapy. The stimulation of vasodilation during such therapy can aid in allowing the chemotherapeutic agent to reach the place that needs treatment. A lot of the unresponsiveness to chemotherapy is due to fact that the drugs never reach the endpoint because of severe vaso-constriction in the surrounding tumour tissue.

In a yet further embodiment of the invention, the individual to be treated is an individual suffering from, or being at risk of developing, acute myocardial infarction. Sympathetic vasoconstriction in the coronary arteries occurs in conditions associated with increases in sympathetic drive and is opposed by metabolic vasodilation. Activation of the purinergic P2Y2 receptors located on the coronary vascular endothelium in conditions associated with coronary vasoconstriction can thus therapeutically be helpful for patents suffering from this disease.

In a yet further embodiment of the use of the invention, the individual to be treated is an individual suffering from, or being at risk of developing, ischemic heart disease.

In an even further embodiment of the use of the invention, the individual to be treated is an individual suffering from, or being at risk of developing, angina pectoris e.g. chest pain due to ischemia (lack of blood and hence oxygen supply) of the heart muscle. Some people with chest pain have normal or minimal narrowing of heart arteries; in these patients, vasospasms referring to a condition in which blood vessels spasm, leading to vasoconstriction, is a likely cause for the pain.

Myocardial ischemia comes about when the myocardium, which is more commonly called as the heart's muscles, fails to take in the much needed blood and oxygen in order to function correctly. The inability to acquire blood and oxygen, on the other hand, is due to blocked or narrowed blood vessels.

In a yet further embodiment of the use of the invention, the individual to be treated is an individual suffering from, or being at risk of developing congestive heart failure because the purinergic stimulation will be positively inotropic and thus increase contraction and at the same time decrease the blood pressure so that the heart can contract better, thus improving this condition.

In an even further embodiment of the use of the invention, the individual to be treated is an individual suffering from, or being at risk of developing, erectile dysfunction.

Hypertension is often associated with diabetes, as at high number of diabetic patients have hypertension and similarly a high number of patients with hypertension have diabetes. There also seems to be a metabolic link between diabetes and high blood pressure due to a resistance in the way the body reacts to insulin, and insulin is not only a good thing, because it causes sugar to go into cells, but it also has some perhaps bad effects in the sense that it causes too much salt retention because of its effect on the kidney.

Hypertension is an important parameter in assessment of other disease and syndromes such as diabetes and metabolic syndrome. The link is double as hypertension is prevalent in diabetes and metabolic syndrome patients and vice versa.

Metabolic syndrome is a combination of medical disorders that affect a large number of people and increases the risk for cardiovascular disease and diabetes. Metabolic syndrome is also known under various other names, such as (metabolic) syndrome X, insulin resistance syndrome and Reaven's syndrome or CHAOS (Australia).

In an embodiment the invention relates to the treatment of an individual suffering from or at risk at acquiring metabolic syndrome or diabetes.

In a further embodiment the invention relates to treatment of an individual suffering from diabetes for the relief of symptoms related to diabetes and complications of diabetes, including, diabetic macroangiopathy, diabetic nephropathy and nephrogenic hypertension. Further conditions linked with increased risk of acquiring diabetes include overweight, obesity, metabolic syndrome X, dysfunctional appetite regulation may be treated according to the invention.

An embodiment the individual to be treated is an individual suffering from diabetes or at risk of developing diabetes. The treatment may according to the invention be a treatment for diabetic complications, particular diabetic complications selected from the group of: diabetic macroangiopathy, diabetic nephropathy, nephrogenic hypertension and diabetic ulcers.

Diabetic ulcers are caused by the combination of arterial blockage and nerve damage. Although diabetic ulcers may occur on other parts of the body they are more common on the foot. The nerve damage or sensory neuropathy reduces awareness of pressure, heat or injury. Rubbing and pressure on the foot goes unnoticed and causes damage to the skin and subsequent 'neuropathic' ulceration. The pathogenesis of diabetic foot ulcers and subsequent infections is complex and involves 3 interactive processes: angiopathy, neuropathy, and immunopathy. Autonomic dysfunction occurs early in the course of neuropathy. In the foot, autonomic dysfunction results in shunting of blood through direct arteriolevenule communications, diminishing the effectiveness of perfusion. Because P2Y2 receptor stimulation overrides autonomic vasoconstriction a profound microcirculatory improvement may be produced and thus diminish the under perfused area whereby diabetic wounds are prevented or treated once they occur.

In a yet further embodiment of the invention the individual to be treated is suffering from diabetic ulcers, particular food ulcers.

### Clinical indications - conditions to be treated with a compound that counteracts P2Y2

As described above, in a further embodiment of the invention, a compound capable of counteracting P2Y2 receptor stimulation is used for the preparation of a medicament for restoring sympathetic vasoconstrictor activity in an individual in need thereof. In one such embodiment, said individual is an individual suffering from, or being at risk of developing, septic shock or cardiogenic shock.

Sepsis results in hyporesponsiveness to α-adrenergic stimulation. This is thought to be mediated by the release of vasoactive compounds from the septic endothelium or by the direct effect of sepsis on vascular smooth muscle (VSM) contractile mechanics and machinery. In such patients increased vasoconstriction is desired. Blockade of the P2Y2 receptor can allow the sympathetic nervous system to restore its activity and cause vasoconstriction through alfa-adrenergic receptor stimulation. ATP discharge from the red blood cells is closely correlated to oxygen discharge from the haemoglobin molecule, thus an increase in ATP discharge from the red blood cells when saturation drops as during septic or cardiogenic shock, can also cause the P2Y2 receptors to be over stimulated. In one embodiment, the measure to prevent release of ATP from the red blood cell is to block the P2Y13 receptor located on the red blood cells, which diminishes the red blood cells ability to excrete ATP. Therefore a P2Y13 blocker is also a suitable treatment for septic shock or cardiogenic shock.

In a preferred further embodiment of all the above embodiments, the individual to be treated is a human being.

### Formulations and mode of administration

The medicament of the invention may be administered in any suitable way known in the art. Preferred modes of administration include parenteral, intravenous, intramuscular, subcutaneous, peroral, such as oral, rectal and intranasal administration. Most preferably, the medicament is administered perorally. Without being bound by one specific theory, it is believed that due to the increased ratio of P2Y2 to P2X1 stimulation as compared to ATP, the undesired effects of P2X stimulation are avoided, even if the compound comes in proximity or in contact with P2X receptor, which e.g. may occur if the compound is administered perorally.

### Compositions for parenteral administration

The compound of the present invention may be formulated for parenteral administration (e.g., by injection, for example bolus injection or continuous infusion) and may be presented in unit dose form in ampoules, pre-filled syringes, small volume infusion or in multi-dose containers with an added preservative. A pharmaceutical composition for parenteral administration may include sterile aqueous and non-aqueous injectable solutions, dispersions, suspensions or emulsions in oily or aqueous vehicles, for example solutions in aqueous polyethylene glycol, as well as sterile powders to be reconstituted in sterile injectable solutions or dispersions prior to use.

The active ingredient may be in powder form, obtained by aseptic isolation of sterile solid or by lyophilisation from solution for constitution before use with a suitable vehicle, e.g., sterile, pyrogen-free water. Aqueous solutions should be suitably buffered if necessary, and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Solutions of compounds or pharmaceutically acceptable salts thereof can be prepared in water or saline, and optionally mixed with a nontoxic surfactant. Compositions for intravenous or intra-arterial administration may include sterile aqueous solutions that may also contain buffers, liposomes, diluents and other suitable additives.

Examples of oily or nonaqueous carriers, diluents, solvents or vehicles for parental use include propylene glycol, polyethylene glycol, animal, synthetic or vegetable oils, and injectable organic esters, and may contain formulatory agents such as preserving, wetting, emulsifying or suspending, stabilizing and/or dispersing agents. Specific examples of oils useful in such compositions include peanut, soybean, sesame, cottonseed, corn, olive, petrolatum, and mineral. Suitable fatty acids for use in parenteral compositions include oleic acid, stearic acid, and isostearic acid. Suitable organic esters include fatty acid esters such as ethyl oleate and isopropyl myristate.

Suitable soaps for use in parenteral compositions include fatty alkali metal, ammonium, and triethanolamine salts, and suitable detergents include (a) cationic detergents such as, for example, dimethyl dialkyl ammonium halides, and alkyl pyridinium halides; (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxy- ethylenepolypropylene copolymers, (d) amphoteric detergents such as, for example, alkyl-beta-aminopropionates, and 2-alkyl-imidazoline quaternary ammonium salts, and (e) mixtures thereof.

The parenteral compositions often will contain from about 0.5 to about 25% by weight of the active ingredient in solution. Preservatives and buffers may be used. In order to minimize or eliminate irritation at the site of injection, such compositions may contain one or more nonionic surfactants having a hydrophile-lipophile balance (HLB) of from about 12 to about 17. The quantity of surfactant in such compositions will typically range from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral compositions can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

The pharmaceutical dosage forms suitable for injection or infusion can include sterile aqueous solutions or dispersions comprising the active ingredient that are adapted for administration by encapsulation in liposomes. In all cases, the ultimate dosage form must be sterile, fluid and stable under the conditions of manufacture and storage.

Sterile injectable solutions can be prepared by incorporating the compound(s) or pharmaceutically acceptable salt(s) thereof in the required amount in the appropriate solvent with various of the other ingredients enumerated above, as required, followed by filter sterilization.

### Compositions for oral delivery

Those compounds that are capable of remaining biologically active in an individual after oral administration can be formulated in a wide range of oral administration dosage forms. The pharmaceutical compositions and dosage forms may comprise the compounds of the invention or its pharmaceutically acceptable salt or a crystal form thereof as the active component. The pharmaceutically acceptable carriers can be either solid or liquid. Solid form preparations include powders, tablets, pills, capsules, cachets, and dispersible granules. A solid carrier can be one or more substances which may also act as diluents, flavouring agents, solubilizers, lubricants, suspending agents, binders, preservatives, wetting agents, tablet disintegrating agents, or an encapsulating material.

Preferably, the composition will be about 0.5% to 75% by weight of a compound or compounds of the invention, with the remainder consisting of suitable pharmaceutical excipients. For oral administration, such excipients include pharmaceutical grades of mannitol, lactose, starch, magnesium stearate, sodium saccharine, talcum, cellulose, glucose, gelatine, sucrose, magnesium carbonate, and the like.

In powders, the carrier can be a finely divided solid which is a mixture with the finely divided active component. In tablets, the active component can be mixed with the carrier having the necessary binding capacity in suitable proportions and compacted in the shape and size desired. The powders and tablets preferably contain 1-70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the composition of the active compound with encapsulating material as carrier providing a capsule in which the active component, with or without carriers, is surrounded by a carrier, which is in association with it. Similarly, cachets and lozenges are included. Tablets, powders, capsules, pills, cachets, and lozenges can be as solid forms suitable for oral administration.

Drops according to the present invention may comprise sterile or non-sterile aqueous or oil solutions or suspensions, and may be prepared by dissolving the active ingredient in a suitable aqueous solution, optionally including a bactericidal and/or fungicidal agent and/or any other suitable preservative, and optionally including a surface active agent. The resulting solution may then be clarified by filtration, transferred to a suitable container which is then sealed and sterilized by autoclaving or maintaining at 98-100 ºC for half an hour. Alternatively, the solution may be sterilized by filtration and transferred to the container aseptically. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Other forms suitable for oral administration include toothpaste, gel dentrifrice orchewing gum. Emulsions may be prepared in solutions in aqueous propylene glycol solutions or may contain emulsifying agents such as lecithin, sorbitan monooleate, or acacia. Aqueous solutions can be prepared by dissolving the active component in water and adding suitable colorants, flavours, stabilizing and thickening agents. Aqueous suspensions can be prepared by dispersing the finely divided active component in water with viscous material, such as natural or synthetic gums, resins, methylcellulose, sodium carboxymethylcellulose, and other well known suspending agents. Solid form preparations include solutions, suspensions, emulsions, syrups and elixirs and may contain, in addition to the active component, colorants, flavors, stabilizers, buffers, artificial and natural sweeteners, dispersants, thickeners, solubilising agents, and the like.

### Compositions for topical administration

The compounds of the invention can also be delivered topically. Regions for topical administration include the skin surface. Compositions for topical administration via the skin and mucous membranes should not give rise to signs of irritation, such as swelling or redness. The compounds described herein can be administered transdermally. Transdermal administration typically involves the delivery of a pharmaceutical agent for percutaneous passage of the drug into the systemic circulation of the patient. The skin sites include anatomic regions for transdermally administering the drug and include the forearm, abdomen, chest, back, buttock, mastoidal area, and the like. The compounds of the present invention may be formulated for topical administration to the epidermis as ointments, creamse, gels or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or colouring agents. Compositions suitable for topical administration in the mouth include lozenges comprising active agents in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

### Compositions for aerosol, nasal or inhalation delivery

The compounds of the present invention may be formulated for administration to the respiratory tract and including intranasal administration. The solutions or suspensions are applied directly to the nasal cavity by conventional means, for example with a dropper, pipette or spray. The compositions may be provided in a single or multidose form. In the latter case of a dropper or pipette this may be achieved by the patient administering an appropriate, predetermined volume of the solution or suspension. In the case of a spray this may be achieved for example by means of a metering atomizing spray pump. A suitable formulation for nasal administration is described in EP 1 466 610.

For inhalation, the compounds of the present invention can be formulated using methods known to those skilled in the art, for example an aerosol, dry powder or solubilised such as in microdroplets, preferably in a device intended for such delivery (such as commercially available from Aradigm, Alkerme or Nektar).

Compositions administered by aerosols may be prepared, for example, as solutions in saline, employing benzyl alcohol or other suitable preservatives, absorption promoters to enhance bioavailability, employing fluorocarbons, and/or employing other solubilising or dispersing agents in accordance with methods known in the art.

### Administration as suppositories

The compounds of the present invention may also be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify. The active compound may be formulated into a suppository comprising, for example, about 0.5% to about 50% of a compound of the invention, disposed in a polyethylene glycol (PEG) carrier (e.g., PEG 1000 [96%] and PEG 4000 [4%].

### Compositions for other types of delivery

Other types of delivery of the compounds according to the present invention are also foreseen, such as implants.

### Formulation

Strategies in formulation development of medicaments and compositions based on the compounds of the present invention generally correspond to methods well-known in the art. E.g. formulation strategies for peptide-based drug products can be dealt with as described in several textbooks, e.g. "Therapeutic Peptides and Protein Formulation. Processing and Delivery Systems", Ed. A.K. Banga, Technomic Publishing AG, Basel, 1995. Injectables are usually prepared either as liquid solutions or suspensions, solid forms suitable for solution in, or suspension in, liquid prior to injection. The preparation may also be emulsified. The active ingredient is often mixed with excipients, which are pharmaceutically acceptable and compatible with the active ingredient. Suitable excipients are, for example, water, saline, dextrose, glycerol, ethanol or the like, and combinations thereof. In addition, if desired, the preparation may contain minor amounts of auxiliary substances such as wetting or emulsifying agents, pH-buffering agents, or which enhance the effectiveness or transportation of the preparation. Some of the compounds of the present invention are sufficiently active, but for some of the others, the effect will be enhanced if the preparation further comprises pharmaceutically acceptable additives and/or carriers. Such additives and carriers will be known in the art. In some cases, it will be advantageous to include a compound, which promotes delivery of the active substance to its target.

### Salts

The active compound may be formulated as neutral or salt forms. Pharmaceutically acceptable salts include acid addition salts (formed with the free amino groups of the peptide compound) and which are formed with inorganic acids such as, for example, hydrochloric or phosphoric acids, or such organic acids as acetic acid, oxalic acid, tartaric acid, mandelic acid, and the like. Salts formed with the free carboxyl group may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

### Dosages

The preparations are administered in a manner compatible with the dosage formulation, and in such amount as will be therapeutically effective. The quantity to be administered depends on the subject to be treated, including, e.g. the weight and age of the subject, the disease to be treated and the stage of disease. Suitable dosage ranges are per kilo body weight normally of the order of several hundred µg active ingredient per administration with a preferred range of from about 0.1 µg to 5000 µg per kilo body weight. Using monomeric forms of the compounds, the suitable dosages are often in the range of from 0.1 µg to 5000 µg per kilo body weight, such as in the range of from about 0.1 µg to 3000 µg per kilo body weight, and especially in the range of from about 0.1 µg to 1000 µg per kilo body weight. Using multimeric forms of the compounds, the suitable dosages are often in the range of from 0.1 µg to 1000 µg per kilo body weight, such as in the range of from about 0.1 µg to 750 µg per kilo body weight, and especially in the range of from about 0.1 µg to 500 µg per kilo body weight such as in the range of from about 0.1 µg to 250 µg per kilo body weight. In particular, when administering nasally smaller dosages are used than when administering by other routes. Administration may be performed once or may be followed by subsequent administrations. The dosage will also depend on the route of administration and will vary with the age and weight of the subject to be treated. A preferred dosage of multimeric forms would be in the interval 1 mg to 70 mg per 70 kg body weight.

In many instances, it will be necessary to administrate the formulation multiple times. Administration may be a continuous infusion, such as intraventricular infusion or administration in more doses such as more times a day, daily, more times a week, weekly, etc. It is preferred that administration of the medicament is initiated before or shortly after the individual has been subjected to the factor(s) that may lead to disease. Preferably the medicament is administered within 8 weeks from the factor onset. Many of the compounds exhibit a long term effect whereby administration of the compounds may be conducted with long intervals, such as 1 week or 2 weeks.

### Examples

### Example 1

This study was performed in nine healthy male subjects from Denmark. The subjects were fully informed of any risks and discomforts associated with the experiments before giving their informed written consent to participate. The studies conformed to the code of Ethics of the World Medical Association (Declaration of Helsinki) and were approved by the Ethics Committee for Copenhagen and Frederiksberg communities.

Subjects reported to the laboratory at 8 a.m. or 1 p.m., following the ingestion of a light breakfast or lunch. Upon arrival, they rested in a supine position while three catheters were placed under local anaesthesia (1% lidocaine) into the femoral artery and vein of the right leg and in the femoral artery of the left leg using the Seldinger technique. The femoral artery and vein catheters were positioned 1-2 cm distal from the inguinal ligament. A thermistor (Edslab probe 94-0.3-2.5F) to measure venous blood temperature was inserted through the femoral venous catheter orientated in the anterograde direction for the determination of femoral venous blood flow. Femoral venous blood flow (an index of leg blood flow (LBF)) was determined by the constant infusion thermodilution technique (Andersen & Saltin, 1985 J Physiol. 366:233-49; González-Alonso et al. 2000 J Physiol. 524:603-15; Am J Physiol Heart Circ Physiol. 278(2):H321-30). LBF represents the average of two measurements made 1-2 min after the start of exercise and 1-2 min after the start of infusion of ATP, adenosine or tyramine. Arterial blood pressure was continuously monitored by a pressure transducer (Pressure Monitoring Kit, Baxter) at the level of inguinal region and calculated by integration of the pressure curve. Heart rate was determined from an electrocardiogram. All data were continuously recorded using a Powerlab system (ADInstruments, Sydney, Australia).

Leg blood flow (LBF) and mean arterial pressure (MAP) was measured during five purinergically-induced hyperaemic conditions: (1) Intrafemoral artery infusion of adenosine (vasodilator control), (2) sequential intraarterial infusion of AMP, ADP, ATP or UTP (vasodilators), and (3), comparing the responses with the combined infusion with the vasoconstrictor drug tyramine, which evokes endogenous release of noradrenaline from sympathetic nerve endings.

Adenosine (Item Development AB, Stocksund, Sweden) was dissolved in isotonic saline (1.25 mg ml⁻¹) and infused at a rate of 16 µmol min⁻¹ AMP (Sigma) was dissolved in isotonic saline and infused at 71 µmol/min, ADP (Sigma) was dissolved in isotonic saline at 44 µmol min⁻¹, ATP (Sigma) was dissolved in isotonic saline at 1 µmol^{/min}and UTP (Sigma) was dissolved in isotonic saline1µmol min⁻¹

The aim to use adenosine as a control condition for the effects of the other drugs was:
1) It targets specifically P1 receptors to induce dilation i.e. a different purinergic vasodilator system.
2) It does not possess the ability to override sympathetic vasoconstriction at rest (Rosenmeier et al. 2003 J Appl Physiol. 95:2370-4; J Physiol. 547:971-6).
   The combined infusion of adenosine and tyramine was to evoke a vasoconstrictor response in the resting leg of about 50%, without causing increases in arterial blood pressure, as previously documented in the Leg (Rosenmeier et al. 2004 J. Physiol. 558:351-365). Tyramine (Sigma T-2879) dissolved in isotonic saline (0.52 mg ml⁻¹) was infused at a rate of about 13.2 µmol/minthrough all the tyramine trials. In separate studies in the resting leg, tyramine infusion at this dose reduced blood flow by 44 ± 3%, without altering mean arterial pressure.

The subjects first received separate infusions of adenosine, AMP, ADP, ATP or UTP for 4 min followed by combined infusions of vasodilator and tyramine for an additional 4 min.

Plasma noradrenaline and adrenaline concentrations were determined with high performance liquid chromatography with electrochemical detection (Hallman et al. 1978 Life Sci. 23(10):1049-52). Arterial and femoral haemoglobin concentration and O₂ saturation were determined spectrophotometrically (OSM-3 Hemoximeter, Radiometer). *P*_{O2} was determined with the Astrup technique (ABL 5, Radiometer, Copenhagen, Denmark).

Leg vascular conductance was calculated as the quotient between LBF and mean arterial pressure. Leg O₂ delivery was calculated by multiplying LBF by arterial O₂ content. Leg O₂ uptake (Leg*^{V̇}*^{O2}) was calculated by multiplying the LBF by the difference in O₂ content between the femoral artery and vein (a-v O₂ difference).

### Statistical analysis

A one-way repeated measures analysis of variance (ANOVA) was performed to test significance between and within treatments. Following a significant *F* test, pair-wise differences were identified using Tukey's honestly significant difference (HSD) *post hoc* procedure. When appropriate, significant differences were also identified using Student's paired *t* tests. The significance level was set at *P <* 0.05. Data are presented as mean ±S.E.M.

### Results:

### Leg haemodynamic responses to tyramine infusion during hyperaemia

In all five hyperaemic conditions, LBF equally increased from approximately 0.5 +/- 0.1 I min(-1) at rest to approximately 3.6 +/- 0.3 I min(-1).Tyramine caused significant leg vasoconstriction during adenosine (56%), AMP(53%) and ADP(30%) infusion, but this was completely abolished by both ATP and UTP infusion (0%).

Maximal UTP-induced vasodilatation was investigated at rest accounted for 50 % 78% of the peak LBF during maximal bicycling exercise in comparison to the 78% increase in LBF previously registered for ATP (Rosenmeier et al. 2004 J. Physiol. 558:351-365).

During pharmacologically induced vasodilatation, LBF increased to similar levels as previously registered during exercise hyperaemia (∼3.6 ±0.3 I min⁻¹) from resting values of ∼0.5 ± 0.1 I min⁻¹, while mean arterial pressure and arterial O₂ content remained unchanged. Hence, the elevations in leg vascular conductance and O₂ delivery during hyperaemia were proportional to the increases in LBF (figure 1).

Fig 1: Blood flow responses to vasodilators and Norepinephrine discharge by TyramineTyramine infusion reduced LBF during adenosine infusion from 3.8± 0.3-to 1.7 ± 0.2 I min⁻¹. During AMP infusion LBF was reduced from 3.7± 0.4 to 1.7± 0.2 I min⁻¹ During ADP infusion LBF was reduced from 3.34± 0.4 to 2.3± 0.3 I min⁻¹, but in contrast, during UTP and ATP infusion, tyramine did not alter either LBF (P= 0.53) or leg vascular conductance.

Reflecting the decline in LBF with combined infusion of tyramine and adenosine, leg a-v O₂ difference increased from 7.7 ± 0.5 ml I⁻¹ with adenosine infusion to 26.0± 2.2 ml I⁻¹ with combined adenosine and tyramine (P < 0.05). Similarly the same happened for AMP alone 4.5± 0.5 ml I⁻¹ to AMP with combined tyramine infusion 15.3±3.1 ml I⁻¹ During ADP infusion a-v difference changed 6.0± 1 ml I⁻¹ to 8.5±-0.8ml I⁻¹, which was not significant (P=0.077), however VO₂ was statistically significantly different.

Absolutely no change in leg a-v O₂ difference was observed with the addition of tyramine during ATP infusion (7.9 ± 1.7 and 5.7 ± 1.6 ml I⁻¹, respectively; *P*= n.s.) or during UTP and tyramine infusion (7.1± 0.9 and 7.1 ± 1.5 ml I⁻¹, respectively; *P*= n.s.)

Femoral venous noradrenaline (epinephrine) concentrations remained elevated during the combined hyperaemia+tyramine (from ∼2 to 3-4 nmol I⁻¹)

During hyperaemia, heart rate was identical with adenosine, AMP, ADP, ATP and UTP (85 ±4 beats min⁻¹) and was increased from resting levels (71±3 beats min⁻¹).

A major finding of the present study is that ATP and UTP infusion in the resting leg fully blunts the effects of increased sympathetic vasoconstrictor activity in a manner similar to that previously observed during exercise (simulating other conditions of increased flow and high sympathetic drive i.e. septic shock), whereas adenosine, AMP and ADP infusion does not.

Despite augmented alfa-adrenoreceptor stimulation evoked by tyramine infusion, no reduction in blood flow or vascular conductance was observed during ATP or UTP infusion, yet a substantial vasoconstrictor response was seen during adenosine, AMP and ADP infusion. Leg a-v O₂ difference was also unchanged with a combined UTP and ATP with combined tyramine infusion but was elevated with a the combined adenosine AMP or ADP and tyramine infusion, such that leg *^{V̇}*^{O2}, was maintained at resting levels in both hyperaemic conditions.

### Example 2

The activity of different compounds is tested in vivo using stable and functional human P2Y₂ and P2X₁ receptor expressing cell lines in 1321N1 astrocytoma.

### Generation of Stable Cell Lines

Human 1321N1 astrocytoma cells are maintained in Dulbecco's modified Eagle's Medium (DMEM) supplemented with 10% fetal bovine serum, 100 units penicillin/ml, 100□g streptomycin/ml, 2□mol glutamine/ml, and 500□g geneticin/ml, and grown at 370C in a humidified atmosphere of 5% CO2 and 95% air. Cells are grown to 60 to 80% confluency in 10cm dishes before transfection. Transfection is performed using Lipofectamine 2000 Reagent (Invitrogen), according to manufacturer's instructions. To generate cell lines stably expressing the P2Y2 and P2X1 receptors, cells are seeded and diluted 2 days after transfection and maintained in DMEM supplemented with 500 µg geneticin/ml. The medium is replaced every 3 days with DMEM containing 500 µg geneticin/ml. Receptor-expressing clones are initially identified by Western Blotting using an anti-P2Y2 or anti-P2X1 antibody and the clones chosen for further study are selected and cultured. Western Blot analysis is used to confirm expression of the individual receptors and functional tests such as calcium assays are used to test the receptor positive clones.

The P2Y2 receptor (Genbank Accession Number NM_176072) is amplified by PCR using the following primers: 5'- CACCATGGCAGCAGACCTGGGCCCCTGGAATGA-3' sense and 5- CTACAGCCGAATGTCCTTAG-3' antisense. The PCR product is initially cloned into pENTR-D-TOPO (Invitrogen) and sequenced before transfer into an expression vector.

The P2X1 receptor (Genbank Accession Number NM_002558) is amplified by PCR with using the primers below. The PCR product is initially cloned into pDONOR and sequenced before transfer into the pCDNA3.2-DEST (Invitrogen) expression vector using the BP clonase reaction.
Sense: 5'-GGGGACAAGTTTGTACAAAAAAGCAGGCTTCACCATGGCACGGCGGTTCCAGGA-3'
Antisense: 5'-GGGGACCACTTTGTACAAGAAAGCTGGGTTCAGGATGTCCTCATGTTCT-3'.

### Calcium Mobilization Assay

The assay is based on the FLIPR Calcium 3 Assay kit from Molecular Devices Corporation.
For the assay, 1321 N1 cells stably transfected with the P2Y2 or P2X1 receptor are seeded overnight in a 96-well plate. Cells are incubated with the FLIPR Calcium 3 Assay Kit for 1 hour at 370C. The compound plate will be prepared with dilutions of various compounds in Hanks Buffer. Samples are run in duplicate with a Molecular Devices Flexstation at room temperature. Cell fluorescence is monitored following exposure to compound and ligand. Increases in intracellular calcium are reported as the maximum fluorescence value after exposure minus the basal fluorescence value before exposure.

### Example 3

In a set of experiments in Denmark, at Rigshospitalet including department of intensive care, department of anaesthesiology and Copenhagen Muscle Research Centre the role of P2Y2 receptors in diseases such as diabetes, hypertension and septic disease is investigated. The efficacy of the compounds are tested in animal studies using pigs in the department of cardiology, Lund, Sweden. Animal studies are further used to test the relevance for angina-like conditions. Hereby we establish the effect of blockade or activation of the above-mentioned receptors in counteracting these diseases.

### Essential Hypertension

The efficiency of nucleotides, e.g. P2Y2 stimulation for blood pressure reduction is investigated

The effect of norepinephrine to restore blood pressure to normal when ATP is in circulation is evaluated, as an indication of a potent role of the P2Y2 receptors in blood pressure reduction in diseases such as sepsis.

### A

Essential hypertension is defined by a blood pressure above 160/100.

Patients with serious concurrent medical illness, Arrhythmia, Congestive heart failure are excluded.

Subjects undergo three different protocols, separated by 10-20 min of rest, to determine:
(1) the systemic vasodilatory effects of ATP, UTP, UDP, ADP adenosine and NO on the blood pressure. All compounds are infused in increasing micromolar concentration.
(2) with combined vasoconstrictor effects of the drug phenyleprine, noradrenaline or metaoxidrine during adenosine-, ATP, UTP; UDP and ADP-induced hyperaemia
(3) maximal vasodilatory effects of ATP, ADP, UDP, UTP and adenosine in increasing concentrations.
(4) maximal vasodilatory effects of ATP,UTP, UDP, ADP and adenosine during intrafemoral infusion in hypertensive patients
(5) with the combined infusion of potassiumblockers (5HD) and tyramine

Resting blood flows are increased in a stepwise manner by infusion of nucleotides (Harvard pumps) until blood flow values matches those obtained during other clinical trials. All nucleotides are purchased commercially, adenosine (Item Development AB, Stocksund, Sweden) dissolved in isotonic saline (1.25 mg/ml) is infused at a rate of 16 µmol/min whereas ATP, ADP, UTP, UDP (Sigma) are dissolved in isotonic saline (1 mg/ml) and are infused in the concentrations mentioned herein. The combined infusion of adenosine and vasoconstrictors are used to evoke a vasoconstrictor response in the resting leg of 50%, without causing increases in arterial blood pressure, as previously documented in the forearm Tyramine (Sigma T-2879) dissolved in isotonic saline (0.52 mg ml-1) is infused at a rate of 13.2 µmo/min through all the tyramine trials.
1) Intravenous infusion of nucleotidies (such as ATP, UTP, ADP and AMP).
   The achieved blood pressure reduction with a blood pressure reduction of > 20 % is measured
2) Dose dependent relationship in comparison to well known hypotensives such as the P1 stimulator (adenosine).
3) Comparison to P2X receptors stimulators.

### B

In pigs intra venous infusions of adenosine, ATP, ADP, AMP, adenosine and nitric oxide is performed to evaluate vasodilator and blood pressure reduction potential as above.

In addition to the procedure outlined above P2Y receptor agonist, Suramin, PPADS and MRS are infused.

To asses whether P1 or P2 receptors are the most powerful we block the receptors with theofyllamine (P1 antagonist) or Suramin (mixed p2 receptor antagonist). This is also be assed locally.

In addition norepinephrine is infused to evaluate whether P2Y2 receptors when stimulated systemically are able to oppose ATP and UTP infusion.

### Ulceration (diabetic)

The benefit for diabetic patients of local P2Y stimulation to increase cutaneous blood flow is investigated. Further investigated are the fibrinolytically benefit from this in the ischemic areas?

Defined by consensus criteria, patients included are diabetic patients that have manifested diabetes 1 with crural leg wounds that need intensive treatment perhaps even surgery.
1) Intraarterial infusion of nucleotides.
   The significance of increase in cutaneous blood flow, above 20%, is measured by laser Doppler flowmetry.
2) Intraarterial infusion of nucleotides.
   The blood flow in skeletal muscle blood flow is measured and compared to blood flow levels achieved during exericse.
3) tPa is measures to determine the effect of purinergic stimulation as a fibrinolytica.

### Septic shock/cardiogenic shock:

Sepsis results in hypo responsiveness to α-adrenergic stimulation. This is thought to be mediated by the release of vasoactive compounds from the septic endothelium or by the direct effect of sepsis on vascular smooth muscle (VSM) contractile mechanics and machinery. In such patients increased vasoconstriction is desired. Blockade of the P2Y2 receptor may allow the sympathetic nervous system to restore its activity and cause vasoconstriction through alpha-adrenergic receptor stimulation. ATP discharge from the red blood cells is closely correlated to oxygen discharge from the haemoglobin molecule, thus an increase in ATP discharge from the red blood cells when saturation drops as during septic or cardiogenic shock, may also cause the P2Y2 receptors to be over stimulated.

Blockage of the P2Y13 receptor located on the red blood cells diminishes the red blood cells ability to excrete ATP. Therefore a P2Y13 blocker may be a suitable treatment for septic shock or cardiogenic shock.
1) Are septic patients increasing their vasodilator capacity by over 20 % in peripheral conductance?
2) Increasing contractility and thus increasing cardiac output by >20 %
3) Is there an Increase in ATP and UTP secretion dependent upon oxygenation? And does this cause the decrease in blood pressure.
4) Is there a reduced vasoconstrictor activity during endotoxemia that worsens by the degree of hypoxia?

This study is designed to establish a relationship between P2Y receptor stimulation during hyper and hypo dynamic phase in sepsis and the therapeutic relevance to block these receptors to maintain pressure during sepsis.

Septic shock are in these studies defined by consensus criteria (APACHE score).

In patients above age 18 we investigate whether there is a reduced vasoconstrictor activity in endotoxemic patients leading to reduced blood pressure and whether this reduced vasopressor response is exaggerated during hypoxia when ATP (and other nucleotides are released) depending on the degree of desaturation on haemoglobin.
1) Does intraarterial infusion of nucleotides result in local vasodilatation despite the vessels are stimulated by tyramine (norepinephrine release) in normal normoxic subjects (control study)
2) Does sympathetic vasoconstriction diminish when the patients are endotoximic without having the intraarterial infusion of nucleotides?
3) Does the degree of vasodilatation increase when the patients are endotoximic and at the same time are hypoxic.
4) Does the degree of cardiac contractility increase in these situations?
5) Do nucleotide levels increase in patients with sepsis in intensive care units?

This study is designed to establish a relationship between P2Y receptor stimulation during hyper and hypo dynamic phase in sepsis and the therapeutic relevance to block these receptors to maintain pressure during sepsis.

We examine this by inserting:
1) Schwann ganz catheters to measure pulmonary pressure in young adults during inhalation of hypoxemic gas. Mean arterial pressure, blood flow to skeletal muscle bed and cardiac contractility. Cardiac Output, Stroke volume and Tissue Doppler velocity.

Resting blood flows are increased in a stepwise manner by infusion of nucleotides (Harvard pumps) until blood flow values matches those obtained during other clinical trials (Rosenmeier et al 2004, J. Physiol. 558:351-365). All nucleotides are purchased commercially, adenosine (Item Development AB, Stocksund, Sweden) dissolved in isotonic saline (1.25 mg/ml) is infused at a rate of 16 µmol/min whereas ATP, ADP, UTP, UDP (Sigma) are dissolved in isotonic saline (1 mg/ml) and are infused in the concentrations mentioned in example 1. The aim during combined infusion of adenosine and vasoconstrictors are used to evoke a vasoconstrictor response in the resting leg of 50%, without causing increases in arterial blood pressure, as previously documented in the forearm Tyramine (Sigma T-2879) dissolved in isotonic saline (0.52 mg/ml) is infused at a rate of 13.2 µmol/min through all the tyramine trials.

With blood samples: Oxygenation including a-v difference, plasma nucleotides, haemoglobin, nitrohemoglobin, norepinephrine, TNF alpha, and tPa.

In older patients we will hemodynamically measure leg blood flow and a-v difference. Through pico catheters in the femoral artery and vein we will assess a-v diff across the leg whilst measuring cardiac contractility and stroke volume. Tissue Doppler+ strain rate/strain to estimate cardiac contractility.

### Metabolic syndrome:

An artificially situation, which should mimic the well known benefits of exercise on the systemic blood pressure and blood flow to the extremities, i.e. P2Y2 stimulation but at rest, is created.

Metabolic syndrome is a disease defined by:
Plasma insulin> 55 pmol/L,C-peptide >700 pmol/L,
with at least one of the following components: Glucose intolerance = F. P-glucose > 6.1 mmol/L, BP>140/90, triclyceride > 2.0 mmol/L and or HDL<1.0 mmol/L, abdominal circumference > 94 cm for men > 80 cm for women, BMI over 30kg/m2

In patients below age 50 we investigate the effect of systemic P2Y2 receptor stimulation during either intraarterial or intra venous infusion of ATP, UTP, Adenosine, on:
1) p-glucose and or p-insulin secretion (a significant change is at least 20 % with regard to start level.)
2) reduce blood pressure and flow variables during ADP, ATP and UTP infusion in comparison to control and during exercise (a significant change is at least 20 % of initial mean arterial pressure and blood flow.)
3) (significantly) alter heart rate without causing arrhythmia.
4) skeletal muscle perfusion (an Improvement of >20%) and increase glucose uptake

Resting blood flows are increased in a stepwise manner by infusion of nucleotides (Harvard pumps) until blood flow values matches those obtained during other clinical trials (Rosenmeier et al. 2004). All nucleotides are purchased commercially, adenosine (Item Development AB, Stocksund, Sweden) dissolved in isotonic saline (1.25 mg ml-1) is infused at a rate of 16 µmol/min whereas ATP,ADP,UTP,UDP (Sigma) are dissolved in isotonic saline (1 mg ml-1) and are infused in the concentrations mentioned in example 1.

The aim during combined infusion of adenosine and vasoconstrictors are used to evoke a vasoconstrictor response in the resting leg of 50%, without causing increases in arterial blood pressure, as previously documented in the forearm Tyramine (Sigma T-2879) dissolved in isotonic saline (0.52 mg ml-1) is infused at a rate of 13.2 µmol/min through all the tyramine trials.These responses are compared to previously tested exercise protocol at 20 W.

In the set up an artificially situation is created, which mimic the well known benefits of exercise on the systemic blood pressure and blood flow to the extremities, ie P2Y2 stimulation but at rest.
Same studies are performed during systemic infusion as seen during essential hypertension but in these protocols also metabolites by-products to glucose metabolism is measured.

### Overview of example 3

| Subjects | Local infusion | Systemic infusion | Experimental set up | Samples/ analysis |
|---|---|---|---|---|
| Healthy | 1) UTP + | ATPmg2+ | +/- ischemia | MAP |
| 1. | a) Phenyl ephrine | ATP | | HR |
| | b) Metaoxidrine | ADP | Applied by a | (Arrhythmia) |
| | c) Clonidine | UTP | blood | Cardiac |
| | d) Prazozin | UDP | pressure cuff, | contractility |
| | e) yohimbin | SNP | before, during | (echo |
| | f) 5-hydroxy-deanaionic acid | Adenosine | or after nucleotide | cardiography) Blood flow |
| | | Norepinphrine | infusion. | |
| | 2) ATP + a-f) as above | | Contra-laterally | |
| | 3) Adenosine + a-f) as above | | (ischemia applied to the either healthy extremity or to the extremity which is not studied.) | |
| 2. | a)-no infusion | | a)-d) | MAP |
| | b) ATP, 30 min | | Ischemia 20 | HR |
| | c) UTP, 30 min | | min. flow | (Arrhythmia) |
| | d) Adenosine, 30 min | | e) contra-lateral infusion | Cardiac contractility |
| | e) no infusion | | | (echo cardiography) Blood flow |
| Hypertension | ATP | ATPmg2+ | +/- ischemia | MAP |
| | UTP | ATP | | HR |
| | adenosine | ADP | As above | (Arrhythmia) |
| | acetylcholine | UTP | | Cardiac |
| | ATP + | UDP | | contractility |
| | theophyllamine | SNP | | (echo |
| | UTP + theophyllamine | Adenosine | | cardiography) Blood flow |
| | ATP + 5HD | Norepinphrine | | (a. femoralis) Maximal |
| | UTP + 5HD | | | dilatatory |
| | | | | Skin |
| Diabetic | ATP | | +/- ischemia | MAP |
| ulceration | UTP | | | HR |
| | UDP | | As above | Cardiac |
| | adenosine | | | contractility |
| | acetylcholine | | | Maximal dilatatory |
| | ATP+ | | | Blood flow: |
| | theophyllamine | | | (a. femoralis) |
| | UTP+ | | | BS: myoglobin |
| | theophyllamine | | | |
| | 5HD | | | |
| Metabolic | ATP | ATP | Glucose | MAP |
| syndrome | UTP | UTP | clamp at | HR |
| | Adenosine | Adenosine | blood glucose | Cardiac |
| | SNO | SNP | 1 and 11 | contractility |
| | Exercise | Exercise | (local infusion) | |
| Pigs | Tyramine Intra coronary | Tyramine ATPmg2+ | +/- ischemia | MAP |
| 1 | infusion of: | ATP | | HR |
| | ATP | ADP | Applied by a | (Arrhythmia) |
| | ADP | UTP | blood | Cardiac |
| | UTP | UDP | pressure cuff | contractility |
| | UDP | SNP | or an | (Thermodilutio |
| | SNF | Adenosine | intraarterial | n) |
| | Adenosine | | ballon | |
| | | Suramin | inserted into | |
| | Suramin | PPADS | the coronary | Blood flow |
| | 5-hydroxy- | 5-hydroxy- | artery. | (via transonic |
| | deanaionic acid | deanaionic | | probe) |
| | Phenyl ephrine | acid | | |
| | Clonidine | ADP | | |
| | Metaoxidrine | (MRS2179) | | |
| | | 1) Phenyl ephrine | | |
| | | 2) Clonidine | | |
| | | 3) Meta-oxidrine | | |
| 2 | Intra coronary infusion of: | | Max. dilatation | MAP HR |
| | ATP | | Coronary flow | (Arrhythmia), |
| | ADP | | reserve | Cardiac |
| | UTP | | +/- coronary | contractility |
| | UDP | | occlusion | (Thermodilutio |
| | ADP | | | n) |
| | SNP Adenosine | | | Blood flow (via transonic probe) |
| 3 | a) no infusion | | a)-d) | Heart infarct |
| | b) ATP, 30 min | | intra-coronary | (Evans blue |
| | c) UTP, 30 min | | ischemia 40 | straining and |
| | d) ADO, 30 min | | min ∼flow | torponins) |
| | e) no infusion | | e) leg ischemia 40 min ∼flow | CKMB |
| Pig pancreas | ATP | ATP | Blod samples: | MAP |
| | ADP | ADP | | BS:insulin, |
| | UTP | UTP | | glucagon, |
| | UDP | UDP | | somatostatin, |
| | Adenosine | Adenosine | | glucose, |
| | MSR2179 | | | noradrenalin |
| | Suramin | alpha,beta- | | Blood flow (a. |
| | Theophyllamin | methylene ATP | | Pacreatica) |
| | alpha,beta-methylene ATP | | | |

| | | | | |
|---|---|---|---|---|
| MAP is mean arterial pressure measure intra-arterial | | | | |

HR is heart rate measured by electrocardiography.

Ischemia is reduced oxygenation in a tissue applied by either a blood pressure cuff or an intra-arterial balloon inserted into the coronary artery. +/- refers to this being applied to a leg or to the coronary circulation before, during or after nucleotide infusion. Contra laterally refers to the condition being applied to the either healthy extremity of to the extremity which is not studied.

Glucose clamping refers to infusion of glucose and insulin to match a certain blood glucose level.

Cardiac contractility is measured by either thermo dilution, blood pressure (dP/dt) or echo cardiography.

Arrhythmia is defined by a cardiac rhythm which is different from sinus rhythm.

Blood flow is measured by cutanous, intra-arterial or transonic Doppler technology

CKMB is P-creatinkinase Isoenzym MB.

## Claims

1. Use of a compound capable of modulating the activation of the P2Y2 receptor pathway for the preparation of a medicament for the treatment of a haemodynamic condition in an individual in need thereof, wherein said compound is a P2Y2 agonist.

2. The use of claim 1, wherein said individual is an individual suffering from, or being at risk of developing, hypertension.

3. The use of claim 2, wherein said hypertension is arterial hypertension, such as primary arterial hypertension.

4. The use of claim 2, wherein said hypertension is pulmonary hypertension, such as primary pulmonary hypertension.

5. The use of claim 1, wherein said individual is an individual suffering from pheochromocytoma.

6. The use of claim 1, wherein said individual is an individual suffering from, or being at risk of developing, erectile dysfunction.

7. The use of claim 1, wherein said individual is an individual undergoing chemotherapy.

8. The use of claim 7, wherein said chemotherapy is anti-neoplastic therapy.

9. The use of claim 1, wherein said individual is an individual suffering from, or being at risk of developing diabetic complications

10. The use of claim 1, wherein said individual is an individual suffering diabetic ulcers.

11. The use of claim 1, wherein said compound is used for the preparation of a medicament for increasing blood flow and reducing augmented sympathetic vasoconstrictor activity in an individual in need thereof.

12. The use of any of the preceding claims, wherein the medicament is for peroral administration.

13. The use according to any of the preceding claims, wherein the compound is UTP.

14. The use according to any of the preceding claims 1-12, wherein the compound is P¹-(uridine 5')-P⁴-(2'-deoxycytidine 5')tetraphosphate or a salt thereof.

15. The use according to claim 14, wherein the compound is the tetrasodium salt of P¹-(uridine 5')-P⁴-(2'-deoxycytidine 5') tetraphosphate (INS37217).

16. A method for identifying a compound capable of increasing blood flow and reducing augmented sympathetic vasoconstrictor activity, said method comprising the steps of:
i) contacting a candidate compound with a functional P2Y2 receptor, and
ii) assessing in a quantitative manner whether or not said compound has a stimulating effect on said P2Y2 receptor.

17. A method for evaluating whether a compound is capable of increasing blood flow and reducing augmented sympathetic vasoconstrictor activity, said method comprising the steps of:
i) contacting a candidate compound with a functional P2Y2 receptor, and
ii) assessing in a quantitative manner whether or not said compound has a stimulating effect on said P2Y2 receptor.

18. A method for evaluating whether a compound is capable of increasing blood flow and reducing augmented sympathetic vasoconstrictor activity, said method comprising the steps of:
i) contacting a candidate compound with a functional P2Y2 receptor, and quantifying the levels of stimulation of said P2Y2 receptor, and
ii) contacting a candidate compound with a functional P2X1 receptor and quantifying the levels of stimulation of said P2X1 receptor evaluating the ratio of EC50 for stimulation of the P2Y2 receptor divided by the EC50 for stimuation of the P2X1 receptor and comparing it with the corresponding ratio for ATP.

19. The method of claim 18 wherein said ratio is at least 2 fold higher for said compound than the corresponding ration for ATP.

20. The method of claim 18, wherein said ratio is at least 10 fold higher for said compound than the corresponding ration for ATP.

21. The ex vivo method of claim 16-20, comprising contacting 132N1 astrocytoma cells stably transfected with the human P2Y2 receptor with the compound to be tested, and assaying the mobilization of intracellular calcium is assayed.

22. The ex vivo method of any of claims 16-21, comprising the further step of assessing in a quantitative manner whether or not said compound has a stimulating effect on the human P2X1 receptor.
